# EUROPEAN PATENT SPECIFICATION

(11) **EP 1 313 521 B1**
(45) Date of publication and mention of the grant of the patent: **11.05.2005**
(21) Application number: 01974978.7
(22) Date of filing: 10.08.2001
(51) Int. Cl.: A61M 5/44

(54) **APPARATUS FOR HEATING BLOOD OR ANOTHER PHYSIOLOGICAL FLUID**
VORRICHTUNG ZUM ERWÄRMEN VON BLUT ODER EINES ANDEREREN PHYSIOLOGISCHEN FLUIDS
APPAREIL DESTINE A CHAUFFER DU SANG OU UN AUTRE FLUIDE PHYSIOLOGIQUE

(30) Priority: 23.08.2000 NL 1015999
(43) Date of publication of application: 28.05.2003
(73) Proprietor: A.J. Van Liebergen Holding B.V., 3831 DP Leusden (NL)
(72) Inventor: BOUHUIJS, Menno, Cornelis, NL-7556 TV Hengelo (NL); RUTGERS, Petrus, Theodorus, NL-7556 BW Hengelo (NL)
(74) Representative: Van Breda, Jacobus
(86) International application number: PCT/NL2001/000596
(87) International publication number: WO 2002/015967

(56) References cited:
- EP-A- 0 138 489
- EP-A- 0 615 763
- WO-A-93/15779
- WO-A-97/46271
- US-A- 4 111 659
- US-B1- 6 261 261

## Description

The invention relates to an apparatus for warming blood or other physiological fluids, comprising a conduit through which the fluid can be conducted, and heating means for warming the fluid flowing through the conduit.

Such an apparatus is known from the international patent application PCT/US96/04737 (WO 96/32080). The apparatus disclosed therein serves for warming blood and has a transparent conduit so that the blood flowing through the conduit is visible. The blood that flows through the conduit is warmed due to the fact that the conduit is fabricated from an electrically conductive polymer material, so that resistance heating of the material, and thus indirectly of the blood, can take place. A drawback of this known apparatus is that the heating is relatively inefficient, that there is a rather long response time, and is consequently difficult to control.

One object of the invention is to realize an improvement with respect to these points, and to realize a more direct warming of the blood or of the physiological fluid to be warmed in general, such as to provide a better controllability of the fluid temperature. A further object of the invention is to provide an apparatus with which the blood or other physiological fluid can the processed hygienically, and with which the development of so-called hot spots in the (blood) stream is avoided, as occurs with direct heating by means of microwave techniques such as, for example, disclosed in the American patent US-A-5,073,167.

A further object of the invention is to avoid problems inherent to heating by applying electrical energy, in particular the risk of current leaking to a patient receiving the warmed blood or other physiological fluid from the apparatus.

From EP-A-0 138 489 an apparatus is known for the irradiation of somatic cells, in particular the treatment of blood cells, with ultraviolet radiation. To this end a tubular construction is used wherein the radiation source is arranged coaxially. The blood fluid is conducted through the tube wherein the fluid is bordered on one side by the radiation source and on the other side by the wall of the tube in which the radiation source is placed.

WO-A-97/46271 discloses an apparatus for warming blood or other physiological fluids, comprising a conduit through which the fluid can be conducted, and heating means for warming the fluid flowing through the conduit, wherein the heating means comprise at least one source of infrared radiation, the conduit is at least partly transparent to the radiation emitted by the infrared source during operation, and wherein the conduit, at its discharge, is provided with a temperature sensor that is coupled to, and feeds a control member.

In this apparatus a separately adjustable unit is used for controlling the temperature of the fluid that is conducted through the conduit.

The apparatus according to the invention is characterized in that the infrared source be adjustable and connected to the control member. Surprisingly, such an infrared source provides a very well controllable, direct warming of the blood or the fluid flowing through the transparent conduit. Because there is no direct contact at all, such heating can take place very hygienically, and because there is no electrical contact with the conduit or the fluid flowing through the conduit, the risk of leakage currents is totally absent.

The fluid flowing through the conduit may be warmed very efficiently in a first preferred embodiment of the apparatus, which is characterized in that the conduit defines a space wherein the infrared source is placed, and in that the conduit, at least at the side facing the infrared source, is transparent to infrared radiation.

A very suitable second embodiment of the apparatus is characterized in that the conduit extends substantially in a plane, in that the infrared source is situated next to the conduit, and in that the conduit, at least at the side of the source, is transparent to infrared radiation. This allows the blood or the fluid to be warmed safely and simply.

The efficiency and controllability is aided in particular by the fact that the conduit extends substantially in the form of a spiral and that its side facing away from the infrared source is provided with an infrared radiation-reflective coating or film. Such an embodiment of the apparatus according to the invention can, moreover, be produced at relatively low costs.

It is further advantageous for the apparatus to be equipped with a flowmeter connected with the control member, in order to be able to accurately adjust the widely varying currents of blood or other physiological fluid that flows through the apparatus.

A first embodiment with which this may be realized is characterized in that the flowmeter comprises a heat source, and at least one temperatures sensor.

A second preferred embodiment is characterized in that the flowmeter comprises two temperature sensors and a heat source positioned between said temperature sensors or near the temperature sensors positioned at the discharge side.

The invention will now be further elucidated with reference to the drawings, which
in Figure 1, schematically show a longitudinal section of a first preferred embodiment of the apparatus according to the invention; and
in Figure 2, show a top view of the conduit that may be used in a second preferred embodiment of the apparatus according to the invention.

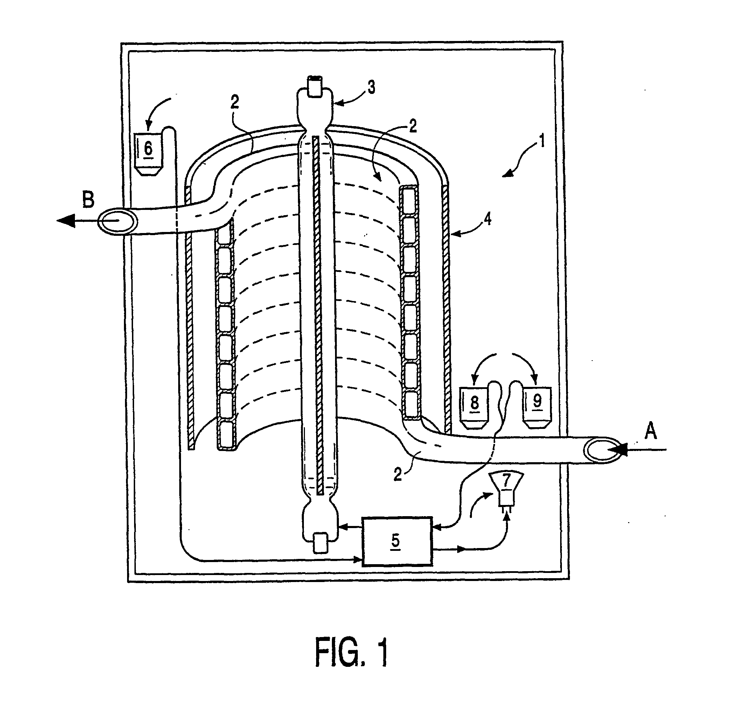

The general workings of the apparatus according to the invention will now first be elucidated with reference to Figure 1.

The apparatus 1 according to the invention for warming blood or other physiological fluids comprises a conduit 2, wherein the entrance is indicated by arrow A and the exit by arrow B. Through this conduit 2 the blood or the physiological fluid to be warmed is conducted by means of propulsion, which in itself is known to a person skilled in the art, and which requires no further elucidation at this point. The Figure shows that the conduit 2 is formed like a spiral, and that in the interior of the spiral shape an infrared source is provided, for example, an infrared lamp 3, which serves to warm the blood or another physiological fluid flowing through the conduit 2. For this purpose the conduit 2 is transparent to infrared radiation, at least at the side facing the infrared lamp 3. It is preferred, however, for the side of the conduit 2 facing away from the infrared lamp 3 to be transparent also, and for this latter side to be provided with a heat-reflective coating or film 4. The infrared lamp 3 is preferably adjustable, being connected to a control member 5. Also connected with the control member 5 is a temperature sensor 6, positioned at the discharge side of the conduit 2. In this way the power supply of the adjustable infrared lamp 3 is rendered dependent on the temperature values of the blood or another physiological fluid that leaves the conduit at the discharge side near arrow B. Conveniently there is also a flowmeter 7, 8, 9 connected to the control member 5, to allow the control member 5 to adjust the power supply of the infrared lamp 3 subject to the amount of blood or other physiological fluid being conducted through the conduit 2. A possible embodiment of the flowmeter 7, 8, 9 is one in which a heat source 7 is used, together with one single temperature sensor 8. The flow rate of the blood or fluid can be determined by, with the aid of the heat source 7, supplying the blood or the other physiological fluid entering the conduit 2 near arrow A with heat pulses at a predetermined known frequency, and by measuring these with the aid of the temperature sensor. The Figure shows another embodiment wherein the flowmeter 7, 8, 9 comprises two temperature sensors 8 and 9, and wherein the heat source 7 is positioned between the temperature sensors 8 and 9. The measured temperature difference between the temperature sensors 8 and 9, together with the amount of heat introduced by the heat source 7, determine the flow rate of the blood or the fluid entering the conduit 2 near the arrow A. It should be noted that the heat source 7 may also be positioned near (opposite to) the temperature sensor 8 near the discharge side.

The foregoing description is to be understood as being a non-limiting exemplary embodiment of the apparatus according to the invention without limiting the protection merited by the appended claims. The example given merely serves to elucidate said claims.

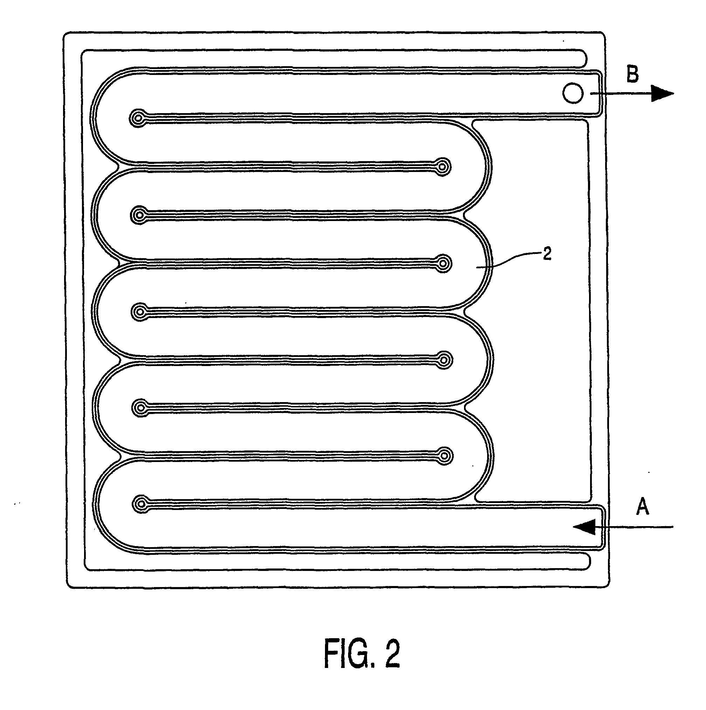

It is, for example, also possible not to embody the conduit 2 as a cylindrical spiral defining a space for housing the infrared lamp 3 but to embody the spiral of the conduit 2 in a plane. A top view of this spiral form of the conduit is shown in Figure 2. Blood or another physiological fluid to be warmed is introduced into the conduit 2 at the entry indicated with arrow A, and leaves the conduit 2 at the exit marked with arrow B. In this embodiment the infrared lamp or lamps are positioned next to the conduit 2 in a manner that is completely obvious to the person skilled in the art, and requires no further elucidation.

In correspondence to the afore-described first preferred embodiment, the conduit 2 is, at least at the side of the lamp 3 or lamps, transparent to infrared radiation, and at the side facing away from the infrared lamp or lamps a heat-reflective coating or film may be provided. The infrared lamp or lamps are adjusted in accordance with the manner described for the first preferred embodiment shown in Figure 1. In practice, said second preferred embodiment, in which the conduit 2 is arranged in a plane is for production technical reasons preferred. The blood or the other physiological fluid conducted through said conduit can be warmed safely and the thus embodied apparatus is mechanically simple to realize.

## Claims

1. An apparatus (1) for warming blood or other physiological fluids, comprising a conduit (2) through which the fluid can be conducted, and heating means (3) for warming the fluid flowing through the conduit (2), wherein the heating means comprise at least one source (3) of infrared radiation, the conduit (2) is at least partly transparent to the radiation emitted by the infrared source (3) during operation, and wherein the conduit (2), at its discharge, is provided with a temperature sensor (6) that is coupled to, and feeds a control member (5), **characterized in that** the infrared source (3) is adjustable and connected to the control member (5).

2. An apparatus according to claim 1 **characterized in that** the conduit defines a space wherein the infrared source is placed, and **in that** the conduit, at least at the side facing the infrared source, is transparent to infrared radiation.

3. An apparatus according to claim 1, **characterized in that** the conduit (2) extends substantially in a plane, **in that** the infrared source (3) is situated next to the conduit (2), and **in that** the conduit (2), at least at the side of the source (3), is transparent.

4. An apparatus according to one of the claims 1-3, **characterized in that** the conduit (2) extends substantially in the form of a spiral and that its side facing away from the infrared source is provided with an infrared radiation-reflective coating or film (4).

5. An apparatus according to one of the preceding claims, **characterized in that** the same is equipped with a flowmeter (7, 8, 9) connected with the control member (5).

6. An apparatus according to claim 5, **characterized in that** the flowmeter (7, 8, 9) comprises a heat source (7) and at least one temperature sensor (8).

7. An apparatus according to claim 5 or 6, **characterized in that** the flowmeter (7, 8, 9) comprises two temperature sensors (8, 9) and a heat source (7) positioned between said temperature sensors (8, 9) or near the temperature sensor (8) positioned at the discharge side.

## Patentansprüche

1. Vorrichtung (1) zum Erwärmen von Blut oder eines anderen physiologischen Fluids, aufweisend: einen Kanal (2), durch welchen das Fluid durchgeleitet werden kann, und eine Erwärmungseinrichtung (3) zur Erwärmung des durch den Kanal (2) strömenden Fluids, wobei die Erwärmungseinrichtung mindestens eine Infrarotstrahlungsquelle (3) aufweist, der Kanal (2) zumindest teilweise gegenüber der durch die Infrarotquelle (3) während des Betriebs emittierte Strahlung durchlässig ist und der Kanal (2) an seinem Auslass mit einem Temperatursensor (6) versehen ist, der mit einem Steuerelement (5) verbunden ist und dieses speist, **dadurch gekennzeichnet, dass** die Infrarotquelle (3) einstellbar ist und mit dem Steuerelement (5) verbunden ist.

2. Vorrichtung nach Anspruch 1, **dadurch gekennzeichnet, dass** der Kanal einen Raum definiert, in welchem die Infrarotquelle platziert ist und dass der Kanal, zumindest an der der Infrarotquelle zugewandten Seite, gegenüber infraroter Strahlung durchlässig ist.

3. Vorrichtung nach Anspruch 1, **dadurch gekennzeichnet, dass** der Kanal (2) sich im Wesentlichen in einer Ebene erstreckt, dass sich die Infrarotquelle (3) in der Nähe des Kanals (2) befindet und dass der Kanal (2) zumindest auf der Seite der Quelle (3) durchlässig ist.

4. Vorrichtung nach einem der Ansprüche 1-3, **dadurch gekennzeichnet, dass** der Kanal (2) sich im Wesentlichen in Form einer Spirale erstreckt und dass seine von der Infrarotquelle weg weisende Seite mit einer infrarote Strahlung reflektierenden Beschichtung oder Film (4) versehen ist.

5. Vorrichtung nach einem der vorhergehenden Ansprüche, **dadurch gekennzeichnet, dass** sie mit einer Strömungsmesseinrichtung (7, 8, 9) ausgerüstet ist, die mit dem Steuerelement (5) verbunden ist.

6. Vorrichtung nach Anspruch 5, **dadurch gekennzeichnet, dass** die Strömungsmesseinrichtung (7, 8, 9) eine Wärmequelle (7) und mindestens einen Temperatursensor (8) aufweist.

7. Vorrichtung nach Anspruch 5 oder 6, **dadurch gekennzeichnet, dass** die Strömungsmesseinrichtung (7, 8, 9) zwei Temperatursensoren (8, 9) und eine Wärmequelle (7) aufweist, die zwischen den Temperatursensoren (8, 9) oder in der Nähe des an der Austrittsseite positionierten Temperatursensors (8) positioniert ist.

## Revendications

1. Appareil (1) pour chauffer du sang, ou d'autres fluides physiologiques, comprenant un conduit (2) à travers lequel le fluide peut être conduit, et des moyens (3) de chauffe destinés à chauffer le fluide s'écoulant à travers le conduit (2),
dans lequel les moyens de chauffe comprennent au moins une source (3) de rayonnement infrarouge, le conduit (2) est au moins partiellement transparent au rayonnement émis par la source infrarouge (3) lorsqu'elle fonctionne, et dans lequel le conduit (2), au niveau de sa section de décharge, est pourvu d'un capteur (6) de température qui est couplé à, et alimente, un élément (5) de commande, **caractérisé en ce que** la source infrarouge (3) peut être réglée et connectée à l'élément (5) de commande.

2. Appareil selon la revendication 1, **caractérisé en ce que** le conduit définit un espace dans lequel est placée la source infrarouge, et **en ce que** le conduit, au moins au niveau du côté qui fait face à la source infrarouge, est transparent au rayonnement infrarouge.

3. Appareil selon la revendication 1, **caractérisé en ce que** le conduit (2) s'étend sensiblement dans un plan, **en ce que** la source infrarouge (3) est située à proximité du conduit (2), et **en ce que** le conduit (2), au moins au niveau du côté qui fait face à la source (3), est transparent.

4. Appareil selon l'une des revendications 1 à 3, **caractérisé en ce que** le conduit (2) s'étend sensiblement en suivant une forme de spirale, et **en ce que** son côté qui fait face à l'écart de la source infrarouge est pourvu d'un revêtement, ou film, réfléchissant un rayonnement infrarouge (4).

5. Appareil selon l'une des revendications précédentes, **caractérisé en ce que** ledit appareil est équipé d'un débitmètre (7, 8, 9) connecté à l'élément (5) de commande.

6. Appareil selon la revendication 5, **caractérisé en ce que** le débitmètre (7, 8, 9) comprend une source (7) de chaleur et au moins un capteur (8) de température.

7. Appareil selon la revendication 5 ou 6, **caractérisé en ce que** le débitmètre (7, 8, 9) comprend deux capteurs (8, 9) de température et une source (7) de chaleur positionnée entre lesdits capteurs (8, 9) de température ou à proximité du capteur (8) de température qui est positionné du côté section de décharge.
